# EUROPEAN PATENT SPECIFICATION

(11) **EP 1 931 358 B1**
(45) Date of publication and mention of the grant of the patent: **13.10.2010**
(21) Application number: 06779251.5
(22) Date of filing: 31.08.2006
(51) Int. Cl.: A61K 31/7068, A61K 31/7072, A61K 31/551, A61P 31/18

(54) **PHARMACEUTICAL COMBINATIONS CONTAINING LAMIVUDINE, STAVUDINE AND NEVIRAPINE**
PHARMAZEUTISCHE KOMBINATIONEN MIT LAMIVUDIN, STAVUDIN UND NEVIRAPIN
COMBINAISONS PHARMACEUTIQUES CONTENANT DE LA LAMIVUDINE, DE LA STAVUDINE ET DE LA NÉVIRAPINE

(30) Priority: 31.08.2005 IN MU10572005
(43) Date of publication of application: 18.06.2008
(73) Proprietor: CIPLA LIMITED, Bombay 400 008 (IN)
(72) Inventor: LULLA, Amar, Maharashtra (IN); MALHOTRA, Geena, Maharashtra (IN)
(74) Representative: Curtis, Philip Anthony
(86) International application number: PCT/GB2006/003229
(87) International publication number: WO 2007/026156

(56) References cited:
- PUTHANAKIT THANYAWEE ET AL: "Efficacy of highly active antiretroviral therapy in HIV-infected children participating in Thailand's National Access to Antiretroviral Program" CLINICAL INFECTIOUS DISEASES, vol. 41, no. 1, July 2005 (2005-07), pages 100-107, XP009075722 ISSN: 1058-4838
- SHALIT PETER ET AL: "Long-term efficacy of a protease inhibitor-sparing, nucleoside reverse transcriptase inhibitor-limiting antiretroviral regimen with nevirapine, lamivudine, and reduced standard stavudine dosage." HIV CLINICAL TRIALS. 2004 JAN-FEB, vol. 5, no. 1, January 2004 (2004-01), pages 62-64, XP009075727 ISSN: 1528-4336
- LANGE JOEP M A: "Efficacy and durability of nevirapine in antiretroviral drug naive patients." JAIDS JOURNAL OF ACQUIRED IMMUNE DEFICIENCY SYNDROMES, vol. 34, no. Supplement 1, September 2003 (2003-09), pages S40-S52, XP009075719 ISSN: 1525-4135
- ESHLEMAN SUSAN H ET AL: "Analysis of human immunodeficiency virus type 1 drug resistance in children receiving nucleoside analogue reverse-transcriptase inhibitors plus nevirapine, nelfinavir, or ritonavir (Pediatric AIDS Clinical Trials Group 377)" JOURNAL OF INFECTIOUS DISEASES, vol. 183, no. 12, 15 June 2001 (2001-06-15), pages 1732-1738, XP009075726 ISSN: 0022-1899
- WIZNIA ANDREW ET AL: "Combination nucleoside analog reverse transcriptase inhibitor(s) plus nevirapine, nelfinavir, or ritonavir in stable antiretroviral therapy-experienced HIV-infected children: Week 24 results of a randomized controlled trial-PACTG 377" AIDS RESEARCH AND HUMAN RETROVIRUSES, vol. 16, no. 12, 10 August 2000 (2000-08-10), pages 1113-1121, XP009075724 ISSN: 0889-2229
- LAURENT CHRISTIAN ET AL: "Effectiveness and safety of a generic fixed-dose combination of nevirapine, stavudine, and lamivudine in HIV-1-infected adults in Cameroon: open-label multicentre trial" LANCET (NORTH AMERICAN EDITION), vol. 364, no. 9428, 3 July 2004 (2004-07-03), pages 29-34, XP009075720 ISSN: 0099-5355
- SHALIT PETER ET AL: "Long-term safety and efficacy of nevirapine, stavudine and lamivudine in a real-world setting" AIDS (HAGERSTOWN), vol. 15, no. 6, 13 April 2001 (2001-04-13), pages 804-805, XP009075721 ISSN: 0269-9370
- RUDOLF VOIGT: "Lehrbuch der pharmazeutischen Technologie" 1984, VEB VERLAG , BERLIN , XP002410136 page 539, paragraph 25.5 the whole document

## Description

### Technical field

The present invention relates to a pharmaceutical composition and a method of inhibiting human immunodeficiency virus (HIV) comprising the preparation and administration of a homogenous combination of lamivudine, stavudine and nevirapine to an HIV infected patient in an amount which achieves antiviral efficacy.

### Background and prior art

A retrovirus designed human immunodeficiency virus (HIV) is the etiological agent of the complex disease that includes progressive destruction of the immune system (acquired immune deficiency syndrome or AIDS) and degeneration of the central and peripheral nervous system.

A common feature of retrovirus replication is the extensive post-translation processing of precursor polyproteins by a virally encoded protease to generate mature viral proteins required for virus assembly and function. Inhibition of this processing prevents the production of normally infectious virus. Literature reports that genetic inactivation of the HIV encoded protease resulted in the production of immature, non-infectious virus particles. These results indicate that inhibition of the HIV protease represents a viable method for the treatment of AIDS and the prevention or treatment of infection by HIV.

Nucleotide sequencing of HIV shows the presence of a Dol gene in one open reading frame [as reported in 'Nature', 313,277(1985) by Ratner, L. et al]. Amino acid sequence homology provides evidence that the Dol sequence encodes reverse transcriptase, an endonuclease and an HIV protease [Toh, H. et al., EMBO J., 4,1267(1985); Power, M. D. et al., Science, 231,1567 (1986); Pearl, L.H. et al., Nature, 329,351(1987)].

US6486183 relates to the field of antivirals and in particular to HIV reverse transcriptase inhibitors and provides novel compounds, pharmaceutical compositions comprising these compounds and methods for the inhibition of HIV employing them.

WO2004087169 relates to an invention which provides for a pharmaceutical composition useful for the treatment or prophylaxis of viral infections comprising nevirapine and at least one antiviral active compound, wherein Base is selected from the group consisting of thymine, cytosine, adenine, guanine, inosine, uracil, 5-ethyluracil and 2,6-diaminopurine, or a pharmaceutically acceptable salt or prodrug thereof, an example of such antiviral active compound being alovudine.

Lamivudine has proven antiviral activity against human immunodeficiency virus (HIV) and other viruses such as hepatitis B. Lamivudine is commercially available from *Glaxo Wellcome Inc* under trade name EPIVIR. Lamivudine and its use against HIV are described in WO 91/17159 and EP 0382526. Crystalline forms of lamivudine are described in WO 92/21676. Combinations of lamivudine with other reverse transcriptase inhibitors, in particular zidovudine, are described in, for example, WO 92/20344, WO 98/18477, and WO99/55372.

US5047407 discloses (2R, cis)-4-ammino-1-(2-liydroxymethyl-1,3-oxathiolau-5yl)-(1H)-pyrimidin-2-one (Epivir - RTM., Lamivudine) and its use in the treatment and prophylaxis of viral infections. Lamivudine has proven antiviral activity against HIV and other viruses such as HBV.

Stavudine, a nucleoside reverse transcriptase inhibitor, and its preparation are disclosed, for example, in US4978655. It is known that stavudine is effective in the treatment of infections caused by retroviruses such as murine leukemia virus and human immunodeficiency virus, i.e. HIV; HTLV III/LAV virus (the AIDS virus).

Stavudine is commercially available from *Bristol Myers Squibb Co.* under the trademark Zerit^{™} for treatment of HIV as described in US 4978655. Methods of preparation of Stavudine are also described in, for example, AU8519701, WO02/20538, US2001039342 and WO01/77103.

The formulations containing stavudine are further described in, for example, US2002002147, WO017/432 FR2794752 and AU4959101.

Nevirapine is commercially available from *Boehringer Ingelheim* under the trademark Viramune for treatment of HIV as described in US 6172059 and US6255481 and in WO02/092095. The earliest known synthesis of nevirapine, by Hargrave et al, is described in US 5366972.

Another patent EA4767 relates to a combination useful for the treatment of viral infections comprising at least one compound wherein the nucleoside analogue is chosen from zidovudine, didanosine, zalcitabine, stavudine or lamivudine and the non-nucleoside reverse transcriptase inhibitor is chosen from nevirapine, delavirdine or efavirenz and wherein the protease inhibitor is chosen from indinavir, nelfinavir, saquinavir or ritonavir. It also deals with a method for the treatment of viral infections comprising administering a therapeutically effective amount of a compound to a subject suffering from an HIV infection.

In Puthanakit Thanyawee et al, "Efficacy of highly active antiretroviral therapy in HIV-infected children participating in Thailand's National Access to Antiretroviral Program", Clinical Infectious Diseases, vol. 41, no. 1, July 2005 (2005-7), pages 100-107, there is described a therapy for HIV using stavudine-lamivudine with nevirapine.

In Laurent Christian et al, "Effectiveness and safety of a generic fixed-dose combination of nevirapine, stavudine and lamivudine, in HIV-1-infected adults in Cameroon: open-lable multicentre trial" Lancet (North American Edition), vol 364, no. 9428, 3 July 2004, pages 29-34, there is described a therapy for HIV using stavudine, nevirapine and lamivudine.

One substantial and persistent problem in the treatment of AIDS has been the ability of the HIV virus to develop resistance to the individual therapeutic agents employed to treat the disease. Thus, a need remains for an efficacious and long lasting therapy for AIDS which lowers HIV viral levels of patients to undetectable levels and raises CD4 cell counts for prolonged periods of time without the development of resistance.

It is an object of the present invention to provide a pharmaceutical composition, which, inter alia, will assist in inhibiting the human immunodeficiency virus (HTV).

The present invention relates to pharmaceutical compositions for treating human immunodeficiency virus (HIV) infections.

### Object

An object of the present invention is to provide a pharmaceutical composition comprising a single dosage unit containing lamivudine, stavudine, and nevirapine, or pharmaceutically acceptable salts or esters thereof, comprising 10-120 mg lamivudine, 1-30 mg stavudine and 25-170 mg nevirapine, wherein said stavudine is separated from said nevirapine and lamivudine within the composition.

A further object of the present invention is to provide a method for the manufacture of the pharmaceutical composition according to the present invention.

A further object of the present invention is to provide a combination therapy as a method to enhance the effectiveness in treating AIDS and to preclude the development of resistance to individual therapeutic agents.

Yet another object of the present invention is to provide a method for treating, reversing, reducing or inhibiting retroviral infections, in particular HIV infections in a human, which includes administering to a mammal a safe and effective amount of the pharmaceutical composition as set out herein.

Yet another object of the present invention, use of a composition for the treatment of viral infections, particularly retroviral infections, which may include human immunodeficiency virus (HIV) infections.

### Summary

The present invention provides a pharmaceutical composition comprising a single dosage unit containing lamivudine, stavudine, together and nevirapine, or pharmaceutically acceptable salts or esters thereof, comprising 10-120 mg lamivudine, 1-30 mg stavudine and 25-170 mg nevirapine, wherein said stavudine is separated from said nevirapine and lamivudine within the composition.

According to the present invention there is also provided a method of manufacturing a pharmaceutical composition as defined above and a pharmaceutically acceptable carrier.

According to the present invention there is also provided combination therapy as a method to enhance the effectiveness in treating AIDS and to preclude the development of resistance to individual therapeutic agents.

### Description

In the present invention, lamivudine, stavudine and nevirapine are combined together for co-administration. This combination therapy is a method to enhance the effectiveness in treating AIDS and to preclude the development of resistance to individual therapeutic agents.

It will be appreciated that the pharmaceutical combinations according to the invention may be combined with further active ingredients.

Examples of such further active ingredients are acyclic nucleosides such as acyclovir, ganciclovir; interferons such as alpha-, beta-and gaunna-interferon; glucuronation inhibitors such as probenecid; nucleoside transport inhibitors such as dipyridamole ; immunomodulators such as interleukin II (IL2) and granulocyte macrophage colony stimulating factor (GM-CSF), erythropoietin, ampligen, thymomodulin, thymopentin, foscarnet, glycosylation inhibitors such as 2-deoxy-D-glucose,castanospermine, 1-deoxynojirimycin; and inhibitors of HIV binding to CD4 receptors such as soluble CD4, CD4 fragments, CD4-hybrid molecules and inhibitors of the HIV aspartyl protease such as L-735, 524.

HIV causes a variety of clinical conditions including acquired immunodeficiency syndrome (AIDS) and chronic neurological disorders. Single drug treatment regimens typically require long term treatment increasing the evidence of unwanted side effects. Moreover, single drug therapies are particularly vulnerable to mutation in the HIV runs, leading to drug resistant variants of HIV.

Multiple drug regimes dramatically improve the treatment of HIV infected patients. This is because one drug will usually cancel out mutations against other drugs. Multiple drug therapies even inhibit replication of HIV viruses for a period of time sufficient to eliminate HIV from the body.

The success of modem multiple drug treatments for HIV often requires strict compliance with a complex treatment regimen that can require the administration of many different drugs per day, administered at precisely times intervals with careful attention to diet. Patient non-compliance is a well-known problem accompanying such complex treatment regimens. Patient non-compliance is an important problem in the treatment of HIV because such non-compliance may lead to the emergence of multiple drug resistant strains of HIV.

This combination therapy in accordance with the invention provides a method to enhance the effectiveness in treating AIDS and to preclude the development of resistance to the individual therapeutic agents.

In general, during alternation therapy, an effective dosage of each agent is administered serially, whereas in combination therapy, an effective dosage of two or more agents are administered together. The dosages will depend on such factors as absorption, biodistribution, metabolism and excretion rates for each drug as well as other factors known to those of skill in the art. It is to be noted that dosage values will also vary with the severity of the condition to be alleviated. It is to be further understood that for any particular subject, specific dosage regimens and schedules should be adjusted over time according to the individual need and the professional judgment of the person administering or supervising the administration of the compositions. Examples of suitable dosage ranges for nevirapine, preferably 3'-deoxy- 3'-fluorothymidine, further NRTIs and other antivirals can be found in the scientific literature. Many examples of suitable dosage ranges for other compounds described herein are also found in the public literature or can be identified using known procedures. These dosage ranges can be modified as desired to achieve a desired result.

The compositions according to the invention may be administered as often as necessary to achieve the desired therapeutic effect. In practice, the compositions may be administered, for example, once, twice, three times or four times per day; other they may be administered less than once per day, for example once every two days or once per week.

The active ingredients can be administered orally in solid dosage forms, such as capsules, tablets and powders, or in liquid dosage forms, such as elixirs, syrups and suspensions. According to the invention, a pharmaceutical composition may include in combination Lamivudine, Stavudine and nevirapine, or pharmaceutically acceptable derivatives thereof as a solid oral dosage form, preferably as a tablet.

The dosage form according to the invention is preferably a dispersible dosage form. Dispersable tablets rapidly disintegrate in cold water (i.e. water of a temperature from about 5°C to about 30°C) to produce a suspension suitable for ingestion. Dispersable tablets have a number of advantages, in particular, they are easy to administer in paediatric applications, and they are easy to manufacture and store. Therefore, the dosage form preferably includes a disintegrant.

The formulations may be prepared by any of the methods well kown in the art of pharmacy. Pharmaceutical formulation suitable for oral administration may conveniently be presented as discrete units such as capsules, including soft gelatin capsules, cachets or tablets each containing a predetermined amount of the active ingredient (s); as a powder or granules. Tablets and capsules for oral administration may contain conventional excipients such as binding agents, fillers, lubricants, disintegrants, or wetting agents. The tablets may be coated according to methods well known in the art.

The combination therapy may include a weight ratio of nevirapine to Lamivudine, ranging from about 6:3 to 4:3, most preferably about 5:3; and the weight ratio of Lamivudine to Stavudine is preferably about 6:1 to 4:1, most preferably about 5:1.

In these embodiments, a pharmaceutically acceptable salt or ester can be substituted for any one or more of the compounds per se. In yet another aspect of these methods, the weight ratio of Nevirapine to Lamivudine and Stavudine can be any one of the weight ratios set forth above.

Exemplifying the invention is a method of preventing infection by HIV, or of treating infection by HIV, or of preventing or treating AIDS, comprising administering to a subject in need thereof a therapeutically effective amount of the compositions described above.

Further exemplifying is the use of Nevirapine and two or more antiretroviral agents selected from nucleoside reverse transcriptase inhibitors such as Stavudine, Lamivudine, Zidovudine or pharmaceutically acceptable derivatives thereof, in the preparation of a medicament for the treatment of infection by HIV and/or for the treatment of AIDS which comprises an effective amount of above mentioned antiretroviral agents or pharmaceutically acceptable derivatives thereof, together or separately. Additionally, included is a process for malting a pharmaceutical composition comprising combination of nucleoside reverse transcriptase inhibitors and nonnucleoside reverse transcriptase inhibitors as mentioned above and a pharmaceutically acceptable carrier.

Exemplifying the invention is a pharmaceutical composition comprising nevirapine, Stavudine and Lamivudine, and pharmaceutically acceptable derivatives thereof, in a pharmaceutically acceptable carrier.

Lamivudine (also known as 3TC) is a synthetic analogue, chemically known as (2R-cis)-4-Amino-1-[2-(hydroxymethyl) 1,3-oxathiolan-5-yl]-2(1H)-pyrimidinone. Lamivudine has also been referred to as (-)-1-[(2R, 5S) 2-(Hydroxymethyl)-1,3-oxathiolan-5-yl] cystosine, (Hydroxymethyl)-1,3-oxathiolan-5-yl] cystosine.

It has been found that Lamivudine exhibits unexpected advantages when used in combination with known inhibitors of HIV replication. In particular, lamivudine shows a better antiviral effect when used in combination with Stavudine.

Stavudine chemically known as (3'-deoxythymidin-2'-ene(3'-deoxy-2',3'-didehydrothymidine), is the synthetic thymidine nucleoside, now well established as an important and useful chemotherapeutic agent for the therapeutic treatment of patients infected with retroviruses.

The chemical name for nevirapine is 11-cyclopropyl-5,11-dihydro-4-methyl-6H-dipyrido [3,2-b : 2', 3'-e] [1,4] diazepin-6-one. Nevirapine is an HIV-1 specific, non-nucleoside, reverse transcriptase inhibitor (NNRTI). Nevirapine is used for treatment of HIV. It is reported to inhibit reproduction of HIV in the body. Nevirapine is used in conjunction with other retroviral agents.

Interestingly, it has been found that nevirapine when used in antiretroviral regimens that includes nucleoside reverse transcriptase inhibitors like lamivudine and stavudine has been found to be very effective.

By means of the pharmaceutical composition in accordance with the invention treatment regimens for HIV and other viruses can be simplified with the goal of enhancing patient compliance by providing a simplified dosage therapy containing a combination of pharmaceutically acceptable amounts of Lamivudine, Stavudine and Nevirapine or pharmaceutically acceptable derivatives thereof.

The phrase 'pharmaceutically acceptable derivative' as used herein is intended to any pharmaceutically acceptable salt, enantiomer, solvent, ester or salt of such ester, or any other compound or mixture which, upon administration to the recipient, is capable of providing (directly or indirectly) the intended active ingredient or any active metabolite or residue thereof.

In the most preferred embodiment, the pharmaceutical composition of the invention employs a combination safe and therapeutically effective amount of two or more therapeutically active agents viz. safe and therapeutically effective amounts of Nevirapine, or 11-cyclopropyl-5,11-dihydro-4-methyl-6H-dipyrido [3,2-b : 2', 3'-e] [1, 4] diazepin-6-one and its pharmaceutically acceptable salts, solvents and derivatives thereof, a safe and therapeutically effective amounts (-) 2', 3'-dioxy, 3'-thyacytidine (Lamivudine) or its pharmaceutically acceptable salts, solvents and derivatives thereof, a safe and therapeutically effective amounts of Stavudine, (3'-deoxythymidin-2'-ene(3'-deoxy-2',3'-didehydrothymidine), or its pharmaceutically acceptable salts, solvents and derivatives thereof along with a safe and effective amount of pharmaceutically acceptable excipients to maintain the composition's homogeneity prior to tablet compression.

The pharmaceutical composition of the present invention conveniently allows administration of a pharmaceutical kit containing three active compounds in tablet dosage forms containing specific dosage ranges for each compound.
- Lamivudine of about 100-300 mg per unit dosage form
- Stavudine of about 30 -100 mg per unit dosage form
- Nevirapine of about 150 - 400mg per unit dosage form.

According to the present invention, the pharmaceutical composition is for paediatric use and dosage regimen is accordingly adjusted. Dosage ranges for each compound for paediatric use is advantageously in the following range:
- Lamivudine of about 10-120 mg more preferably about 60mg per unit dosage form
- Stavudine of about 1 - 30 mg more preferably about 12mg per unit dosage form
- Nevirapine of about 25 - 170 mg, more preferably 50 - 170 mg, more preferably about 100mg per unit dosage form. ,

Therefore, according to another aspect of the invention there is provided a pharmaceutical composition comprising lamivudine, stavudine, and nevirapine, or pharmaceutically acceptable salts or esters thereof, for separate, simultaneous or sequential administration, comprising 10-120 mg lamivudine, 1-30 mg stavudine and 25-170 mg nevirapine.

Preferably, the pharmaceutical composition comprises 10-100 mg lamivudine, 1-25 mg stavudine and 25-170 mg nevirapine.

Preferably, the wt% of nevirapine in the composition is from 0.75 to 2.0 times, more preferably 0.75 to 1.5 times, the wt% of lamivudine in the composition. Preferably, the wt% of lamivudine in the composition is from 2 to 6 times, the wt% of stavudine in the composition.

The pharmaceutical composition is most preferably a single unit dosage form containing said lamivudine, stavudine and nevirapine. The dosage form is desirably a solid oral dosage form.

One particularly preferred composition comprises a tablet containing 12mg stavudine, 60mg lamivudine and 100mg nevirapine. This is referred to as tablet 1 in the table below.

Another particularly preferred composition comprises a second tablet containing 6mg stavudine, 30mg lamivudine and 50mg nevirapine. This is referred to as tablet 2 in the table below.

This composition is particularly useful for paediatric use i.e for treating human under the age of 16 years. The preferred dosage depends on the body weight of the child to be treated. The following dosages are most suitable:

| Body Weight/kg | Stavudine/mg | lamivudine/mg | Nevirapine/mg | Number of Tablet 1 | Number of Tablet 2 |
|---|---|---|---|---|---|
| 3-5 | 3 | 15 | 25 | | 0.5 |
| 6-7 | 6 | 30 | 50 | | 1 |
| 8-9 | 9 | 45 | 75 | | 1.5 |
| 10-14 | 12 | 60 | 100 | 1 | |
| 15-19 | 15 | 75 | 125 | | 2.5 |
| 20-24 | 18 | 90 | 150 | 1.5 | 3 |
| 25-29 | 24 | 120 | 200 | 2 | |

Compatibility was another aspect which had to be sorted for these 3 drugs. Study was designed so as to evaluate the physico-chemical parameters of these three drugs. Each active ingredients was homogeneously mixed with the other and after a period of four weeks exposure to 25°C/60% RH & 40°C/75% RH , this mixture was analysed.

The details are enclosed below:

| Ingredient | Appearance | | Impurities | |
|---|---|---|---|---|
| | Initial | End of study | Initial | End of study |
| Lamivudine + Nevirapine | White | White | 0.54% | 0.58% |
| Lamivudine + Stavudine | White | Brown | 0.87% | 1.25% |
| Stavudine + Nevirapine | White | Brown | 0.95% | 1.97% |
| Lamivudine + Nevirapine + stavudine | White | Brown | 1.28% | 12.35% |

Lanivudine was compatible with Nevirapine but not with Stavudine. Similarly, stavudine was incompatible with nevirapine.

Incompatibility with Lamivudine & Nevirapine can be avoided by separating stavudine from the other two drugs by one of the following ways
i. Coating of Stavudine or Stavudine granules by suitable film forming polymers & adding to granules at lubrication stage
ii. By employing bilayer/trilayer design for the tablets

The tablet according to the present invention may be prepared by using standard methods of tablet manufacture i.e. wet granulation or direct compression. Coating techniques for the stavudine granules employs methods and equipments which are extensively documented in literature.

The formulation may further comprise binders, diluents, disintegrants, glidants, lubricants and artificial colours. The binders are usually used the ranges of 0.5 to 25%, disintegrants in the range of 0.5 - 25%, lubricants in the range of 0.25% - 10%.

A tablet may also contain some pharmaceutically acceptable fillers as excipients. Examples of suitable fillers/diluents are starch and derivatives, lactose, mannitol, sucrose, glucose, Sorbitol, calcium phosphates, maltodextrins, polyvinylpyrrolidone, polyethylene glycols, microcrystalline cellulose, etc.

The tablets according to the present invention may also contain other excipients like binders (microcrystalline cellulose, starches, polyvinylpyrrolidone and the like), disintegrants (microcrystalline cellulose, sodium starch glycollate, starch, croscarmellose sodium, hydroxypropyl cellulose, etc.), lubricants (talc, Magnesium stearate, colloidal silica and the like), flavouring or colouring agents.

### i. Option I

Stavudine API as a powder or in form of granules may be coated using suitable film forming polymers such as HPMC, HPMCP , Na-CMC HPC, PVA, PVP , acrylates such as Eugragit to E-100, L- 100 , L10055, HPMC acetyl succinate , Xanthan gum etc.

Coated Stavudine may be blended with granules of Lamivudine & Nevirapine along with lubricants & compressed using suitable tooling.

### ii. Option II

a. To manufacture bilayered tablet or trilayeral where in stavudine is incorporated as one of the layer & lamivudine & Nevirapine in the 2^{nd} layer or as a trilayered tablet where in Stavudine layer & lamivudine & Nevirapine layer separated totally by an inert layer.
b. Tablet in a tablet is another option available where in Stavudine layer constitutes inner tablet which may be film coated or uncoated

Therefore, in a preferred embodiment, a barrier is provided between said stavudine and said nevirapine and lamivudine.

In one preferred embodiment, said stavudine is provided in the form of particles coated with a material to prevent contact between said stavudine and said nevirapine and lamivudine.

In another preferred embodiment the pharmaceutical composition comprises two layers, wherein the first layer contains said nevirapine and lamivudine in combination with a pharmaceutically acceptable carrier, and the second layer contains said stavudine in combination with a pharmaceutically acceptable carrier.

In another preferred embodiment the pharmaceutical composition comprises three layers, wherein the first layer contains said nevirapine and lamivudine in combination with a pharmaceutically acceptable carrier, the second layer contains said stavudine in combination with a pharmaceutically acceptable carrier, and the third layer is an inert layer containing at least one pharmaceutically acceptable excipient, wherein the third layer is disposed between the first and second layers.

In another preferred embodiment the pharmaceutical composition comprises a core and an outer layer surrounding the core, wherein the core contains said nevirapine and lamivudine in combination with a pharmaceutically acceptable carrier, and outer layer contains said stavudine in combination with a pharmaceutically acceptable carrier.

In another preferred embodiment the pharmaceutical composition comprises a core and an outer layer surrounding the core, wherein the core contains said stavudine in combination with a pharmaceutically acceptable carrier, and outer layer contains said nevirapine and lamivudine in combination with a pharmaceutically acceptable carrier.

According to another aspect of the invention there is provided the use of a lamivudine, nevirapine and stavudine in the manufacture of a medicament for treatment of a viral infection in a human, wherein said medicament contains 10-120 mg lamivudine, 1-30 mg stavudine and 25-170 mg nevirapine.

The viral infection may be a retroviral infection, such as HIV infection.

According to another aspect of the invention, there is provided a method of treating a viral infection in a human, comprising administering to a human in need thereof an amount of a medicament containing 10-120 mg lamivudine, 1-30 mg stavudine and 25-170 mg nevirapine at therapeutically acceptable intervals.

According to another aspect of the invention, there is provided a method of making a pharmaceutical composition comprising combining 10-120 mg lamivudine, 1-30 mg stavudine and 25-170 mg nevirapine with a pharmaceutically acceptable carrier.

In one aspect, the invention provides a way of providing a single, stable, oral dosage form comprising stavudine, lamivudine and nevirapine.

According to another aspect of the invention there is provide a pharmaceutical composition in the form of an oral dosage form comprising stavudine, lamivudine and nevirapine, wherein the lamivudine and nevirapine are provided in one layer of the oral dosage form, and the stavudine is provided in a separate layer. The dosage form can be provided in the bilayer or trilayer form described above, and may be made by the methods described above.

According to another aspect of the invention there is provided a pharmaceutical composition in the form of an oral dosage form comprising stavudine, lamivudine and nevirapine, wherein said stavudine is provided in the form of particles coated with a material to prevent contact between said stavudine and said nevirapine and lamivudine.

The following examples are for the purpose of illustration of the invention only and are not intended in any way to limit the scope of the present invention.

### Examples 1 :

| **Ingredients** | **Qty** |
|---|---|
| | **(mg/tablet)** |
| Stavudine | 12.00 |
| HPMC | 3.00 |
| Talc | 1.00 |
| Purified water | q.s. |
| Nevirapine | 100.00 |
| Lamivudine | 60 |
| Microcrystalline cellulose(Avicel PH 101) | 237.50 |
| Sodium starch glycollate | 25.00 |
| Starch | 10.00 |
| Sodium starch glycollate | 10.00 |
| Magnesium stearate | 7.50 |

### Examples 2 :

| **Ingredients** | **Qty** |
|---|---|
| | **(mg/tablet)** |
| Stavudine | 12.00 |
| HPC-L | 2.00 |
| Propylene glycol | 0.2 |
| Purified water | q.s. |
| Nevirapine | 100.00 |
| Lamivudine | 60 |
| Microcrystalline cellulose(Avicel PH 101) | 237.50 |
| Sodium starch glycollate | 25.00 |
| Starch | 10.00 |
| Sodium starch glycollate | 10.00 |
| Magnesium stearate | 7.50 |

### Examples 3 :

| **Ingredients** | **Qty** |
|---|---|
| | **(mg/tablet)** |
| Stavudine | 12.0 |
| Eudragit L100 | 7.0 |
| Dibutyl phthalate | 0.7 |
| Isopropyl alcohol | q.s. |
| Purified water | q.s. |
| Nevirapine | 100.00 |
| Lamivudine | 60 |
| Microcrystalline cellulose (Avicel PH 101) | 237.50 |
| Sodium starch glycollate | 25.00 |
| Starch | 10.00 |
| Sodium starch glycollate | 10.00 |
| Magnesium stearate | 7.50 |

### Examples :

### 1. Trilayered :

| **Ingredients** | **Qty (mg/tablet)** |
|---|---|
| **Part I** | |
| Lamivudine | 60.00 |
| Nevirapine | 100.00 |
| Microcrystalline cellulose | 73.00 |
| Starch | 6.00 |
| Sodium Starch glycollate | 8.00 |
| Talc | 1.00 |
| Magnesium Stearate | 2.00 |
| Purified water | q.s. |
| | **250.00** |

| **Part II** | |
|---|---|
| Microcrystalline cellulose | 69.95 |
| Starch | 4.00 |
| Sodium Starch glycollate | 4.00 |
| Colourant | 0.05 |
| Purified water | q.s. |
| Talc | 1.00 |
| Magnesium stearate | 1.00 |
| | **80.00** |

| **Part III** | |
|---|---|
| Stavudine | 12.00 |
| Mannitol | 81.0 |
| Hydroxypropyl cellulose | 4.00 |
| Aerosil | 0.6 |
| Talc | 1.2 |
| Magnesium Stearate | 1.2 |
| | **100.00** |
| **Total** | **430.0 mg** |

### 2. Bilayered.

| **Ingredients** | **Qty(mg/tablet)** |
|---|---|
| **Part - I** | |
| Lamivudine | 60.00 |
| Nevirapine | 100.00 |
| Microcrystalline cellulose | 94.95 |
| Starch | 8.00 |
| Sodium starch glycollate | 10.00 |
| colourant | 0.05 |
| Talc | 3.00 |
| Magnesium stearate | 4.00 |
| Purified water | q.s |

| **Part - II** | |
|---|---|
| Stavudine | 12.00 |
| Mannitol | 81.00 |
| Hydroxypropyl cellulose | 4.00 |
| Colloidal silicon dioxide | 0.60 |
| Talc | 1.20 |
| Magnesium stearate | 1.20 |
| Total | 380.00 |

### b) Tablet in Tablet :

| **Ingredients** | **Qty (mg/tablet)** |
|---|---|
| **Tablet I** | |
| Stavudine | 12.00 |
| Mannitol | 63.95 |
| Hydroxypropyl cellulose | 5.00 |
| Aerosil | 0.5 |
| Talc | 1.0 |
| Magnesium stearate | 2.5 |
| Colourant | 0.05 |
| | **85.00** |

| **Tablet II** | |
|---|---|
| Lamivudine | 60.00 |
| Nevirapine | 100.00 |
| Microcrystalline cellulose | 167.00 |
| Starch | 6.00 |
| Sodium starch glycollate | 8.0 |
| Purified water | q.s. |
| Talc | 1.0 |
| Magnesium stearate | 3.0 |
| | **345.00** |

## Claims

1. A pharmaceutical composition comprising a single dosage unit containing lamivudine, stavudine, and nevirapine, or pharmaceutically acceptable salts or esters thereof, comprising 10-120 mg lamivudine, 1-30 mg stavudine and 25-170 mg nevirapine, wherein said stavudine is separated from said nevirapine and lamivudine within the composition.

2. A pharmaceutical composition according to claim 1, comprising 10-100 mg lamivudine, 1-25 mg stavudine and 25-170 mg nevirapine, preferably 60 mg lamivudine, 12 mg stavudine and 100 mg, nevirapine, more preferably 30 mg lamivudine, 6 mg stavudine and 50 mg nevirapine

3. A pharmaceutical composition according to claim 1 or 2, wherein the wt% of nevirapine in the composition is from times 0.75 to 2.0 times the wt% of lamivudine in the composition, and/or the wt% of lamivudine in the composition is from times 2 to 6 times the wt% of stavudine in the composition.

4. A pharmaceutical composition according to claim 3, wherein said dosage form is an oral dosage form, preferably a solid dosage form.

5. A pharmaceutical composition according to claim 4, wherein a barrier is provided between said stavudine and said nevirapine and lamivudine.

6. A pharmaceutical composition according to claim 4 or 5, wherein said stavudine is provided in the form of particles coated with a material to prevent contact between said stavudine and said nevirapine and lamivudine.

7. A pharmaceutical composition according to claim 4 or 5, comprising two layers, wherein the first layer contains said nevirapine and lamivudine in combination with a pharmaceutically acceptable carrier, and the second layer contains said stavudine in combination with a pharmaceutically acceptable carrier.

8. A pharmaceutical composition according to claim 4 or 5, comprising three layers, wherein the first layer contains said nevirapine and lamivudine in combination with a pharmaceutically acceptable carrier, the second layer contains said stavudine in combination with a pharmaceutically acceptable carrier, and the third layer is an inert layer containing at least one pharmaceutically acceptable excipient, wherein the third layer is disposed between the first and second layers.

9. A pharmaceutical composition according to claim 4 or 5, comprising a core and an outer layer surrounding the core, wherein the core contains said nevirapine and lamivudine in combination with a pharmaceutically acceptable carrier, and outer layer contains said stavudine in combination with a pharmaceutically acceptable carrier.

10. A pharmaceutical composition according to claim 4 or 5, comprising a core and an outer layer surrounding the core, wherein the core contains said stavudine in combination with a pharmaceutically acceptable carrier, and outer layer contains said nevirapine and lamivudine in combination with a pharmaceutically acceptable carrier.

11. A pharmaceutical composition according to any one of claims 4 to 10, wherein said dosage form is a dispersible dosage form.

12. The use of a lamivudine, nevirapine and stavudine in the manufacture of a medicament for treatment of a viral infection in a human, wherein said medicament is in the form of a single dosage unit containing 10-120 mg lamivudine, 1-30 mg stavudine and 25-170 mg nevirapine, and wherein said stavudine is separated from said nevirapine and lamivudine within the medicament.

13. The use according to claim 12, to treat a human under 16 years old.

14. The use of stavudine, lamivudine and nevirapine in the manufacture of a medicament for treating a child suffering from a viral infection and having a weight in the range 3 to under 6 kg, wherein the medicament is in the form of a single dosage unit containing 2 to 4mg stavudine, 10 to 20mg lamivudine and 20 to 30mg nevirapine, preferably 3mg stavudine, long lamivudine and 25mg nevirapine, and wherein said stavudine is separated from said nevirapine and lamivudine within the medicament.

15. The use of stavudine, lamivudine and nevirapine in the manufacture of a medicament for treating a child suffering from a viral infection and having a weight in the range 6 to under 8 kg, wherein the medicament is in the form of a single dosage unit containing 5 to 7mg stavudine, 20 to 40mg lamivudine and 40 to 60mg nevirapine, preferably 6mg stavudine, 30mg lamivudine and 50mg nevirapine, and wherein said stavudine is separated from said nevirapine and lamivudine within the medicament.

16. The use of stavudine, lamivudine and nevirapine in the manufacture of a medicament for treating a child suffering from a viral infection and having a weight in the range 8 to under 10 kg, wherein the medicament is in the form of a single dosage unit containing 8 to 10mg stavudine, 35 to 55mg lamivudine and 65 to 85mg nevirapine, preferably 9mg stavudine, 45mg lamivudine and 75mg nevirapine, and wherein said stavudine is separated from said nevirapine and lamivudine within the medicament.

17. The use of stavudine, lamivudine and nevirapine in the manufacture of a medicament for treating a child suffering from a viral infection and having a weight in the range 10 to under 15kg. wherein the medicament is in the form of a single dosage unit containing 11 to 13mg stavudine, 50 to 70mg, lamivudine and 90 to 110mg nevirapine, preferably 12mg stavudine, 60mg lamivudine and 100mg nevirapine, and wherein said stavudine is separated from said nevirapine and lamivudine within the medicament.

18. The use of stavudine, lamivudine and nevirapine in the manufacture of a medicament for treating a child suffering from a viral infection and having a weight in the range 15 to under 20 kg, wherein the medicament is in the form of a single dosage unit containing 14 to 16mg stavudine, 65 to 85mg lamivudine and 115 to 135mg nevirapine, preferably 15mg stavudine, 75mg lamivudine and 125mg nevirapine, and wherein said stavudine is separated from said nevirapine and lamivudine within the medicament.

19. The use of stavudine, lamivudine and nevirapine in the manufacture of a medicament for treating a child suffering from a viral infection and having a weight in the range 20 to 29 kg, wherein the medicament is in the form of a single dosage unit containing 17 to 19mg stavudine, 80 to 100mg lamivudine and 140 to 160mg nevirapine, preferably 18mg stavudine, 90mg lamivudine and 150mg nevirapine, and wherein said stavudine is separated from said nevirapine and lamivudine within the medicament.

20. The use of stavudine, lamivudine and nevirapine in the manufacture of a medicament for treating a child suffering from a viral infection and having a weight in the range 20 to 24 kg, wherein the medicament is in the form of a single dosage unit containing 18mag stavudine, 90mg lamivudine and 150 mg nevirapine, and wherein said stavudine is separated from said nevirapine and lamivudine within the medicament.

21. The use of stavudine, lamivudine and nevirapine in the manufacture of a medicament for treating a child suffering from a viral infection and having a weight in the range 25-29 kg, therein the medicament is in the form of a single dosage unit containing 23 to 25mg stavudine, 110 to 130mg, lamivudine and 190 to 210mg nevirapine, preferably 24mg stavudine, 120mg lamivudine and 200mg, nevirapine, and wherein said stavudine is separated from said nevirapine and lamivudine within the medicament.

22. The use according to any of claims 16 to 21, wherein the viral infection is a retroviral infection, such as an HIV infection.

23. A pharmaceutical composition in the form of an oral unit dosage form comprising stavudine, lamivudine and nevirapine, wherein the lamivudine and nevirapine are provided in one layer of the oral dosage form, and the stavudine is provided in a separate layer.

24. A pharmaceutical composition in the form of an oral unit dosage form comprising stavudine, lamivudine and nevirapine, wherein said stavudine is provided in the form of particles coated with a material to prevent contact between said stavudine and said nevirapine and lamivudine.

## Patentansprüche

1. Pharmazeutische Zusammensetzung, umfassend eine einzelne Dosiereinheit, die Lamivudin, Stavudin und Nevirapin oder pharmazeutisch verträgliche Salze oder Ester davon enthält, die 10 bis 120 mg Lamivudin, 1 bis 30 mg Stavudin und 25 bis 170 mg Nevirapin umfasst, worin das Stavudin vom Nevirapin und Lamivudin in der Zusammensetzung getrennt ist.

2. Pharmazeutische Zusammensetzung nach Anspruch 1, umfassend 10 bis 100 mg Lamivudin, 1 bis 25 mg Stavudin und 25 bis 170 mg Nevirapin, bevorzugt 60 mg Lamivudin, 12 mg Stavudin und 100 mg Nevirapin, bevorzugter 30 mg Lamivudin, 6 mg Stavudin und 50 mg Nevirapin.

3. Pharmazeutische Zusammensetzung nach Anspruch 1 oder 2, worin der gewichtsprozentige Anteil von Nevirapin in der Zusammensetzung von 0,75- bis 2,0-mal den gewichtsprozentigen Anteil von Lamivudin in der Zusammensetzung beträgt, und/oder der gewichtsprozentige Anteil von Lamivudin in der Zusammensetzung von 2- bis 6-mal den gewichtsprozentigen Anteil von Stavudin in der Zusammensetzung beträgt.

4. Pharmazeutische Zusammensetzung nach Anspruch 3, worin die Dosierungsform eine orale Dosierungsform, bevorzugt eine feste Dosierungsform ist.

5. Pharmazeutische Zusammensetzung nach Anspruch 4, worin ein Barriere zwischen dem Stavudin und dem Nevirapin und Lamivudin bereitgestellt ist.

6. Pharmazeutische Zusammensetzung nach Anspruch 4 oder 5, worin das Stavudin in der Form von Partikeln bereitgestellt ist, die mit einem Material zur Verhinderung des Kontakts zwischen dem Stavudin und dem Nevirapin und Lamivudin beschichtet sind.

7. Pharmazeutische Zusammensetzung nach Anspruch 4 oder 5, umfassend zwei Schichten, worin die erste Schicht das Nevirapin und Lamivudin in Kombination mit einem pharmazeutisch verträglichen Träger enthält, und die zweite Schicht das Stavudin in Kombination mit einem pharmazeutisch verträglichen Träger enthält.

8. Pharmazeutische Zusammensetzung nach Anspruch 4 oder 5, umfassend drei Schichten, worin die erste Schicht das Nevirapin und Lamivudin in Kombination mit einem pharmazeutisch verträglichen Träger enthält, die zweite Schicht das Stavudin in Kombination mit einem pharmazeutisch verträglichen Träger enthält, und die dritte Schicht eine inerte Schicht mit mindestens einem pharmazeutisch verträglichen Hilfsstoff ist, worin die dritte Schicht zwischen den ersten und zweiten Schichten angeordnet ist.

9. Pharmazeutische Zusammensetzung nach Anspruch 4 oder 5, umfassend einen Kern und eine den Kern umgebende Außenschicht, worin der Kern das Nevirapin und Lamivudin in Kombination mit einem pharmazeutisch verträglichen Träger enthält, und die Außenschicht das Stavudin in Kombination mit einem pharmazeutisch verträglichen Träger enthält.

10. Pharmazeutische Zusammensetzung nach Anspruch 4 oder 5, umfassend einen Kern und eine den Kern umgebende Außenschicht, worin der Kern das Stavudin in Kombination mit einem pharmazeutisch verträglichen Träger enthält, und die Außenschicht das Nevirapin und Lamivudin in Kombination mit einem pharmazeutisch verträglichen Träger enthält.

11. Pharmazeutische Zusammensetzung nach einem der Ansprüche 4 bis 10, worin die Dosierungsform eine dispergierbare Dosierungsform ist.

12. Verwendung eines Lamivudins, Nevirapins und Stavudins in der Herstellung eines Arzneimittels zur Behandlung einer Virusinfektion in einem Menschen, worin das Arzneimittel in der Form einer einzelnen Dosiereinheit mit 10 bis 120 mg Lamivudin, 1 bis 30 mg Stavudin und 25 bis 170 mg Nevirapin vorliegt, und worin das Stavudin vom Nevirapin und Lamivudin im Arzneimittel getrennt ist.

13. Verwendung nach Anspruch 12, zur Behandlung eines unter 16 Jahre alten Menschen.

14. Verwendung von Stavudin, Lamivudin und Nevirapin in der Herstellung eines Arzneimittels zur Behandlung eines an einer Virusinfektion leidenden Kindes und mit einem Gewicht im Bereich von 3 bis unter 6 kg, worin das Arzneimittel in der Form einer einzelnen Dosiereinheit mit 2 bis 4 mg Stavudin, 10 bis 20 mg Lamivudin und 20 bis 30 mg Nevirapin, bevorzugt 3 mg Stavudin, 15 mg Lamivudin und 25 mg Nevirapin vorliegt, und worin das Stavudin vom Nevirapin und Lamivudin im Arzneimittel getrennt ist.

15. Verwendung von Stavudin, Lamivudin und Nevirapin in der Herstellung eines Arzneimittels zur Behandlung eines an einer Virusinfektion leidenden Kindes und mit einem Gewicht im Bereich von 6 bis unter 8 kg, worin das Arzneimittel in der Form einer einzelnen Dosiereinheit mit 5 bis 7 mg Stavudin, 20 bis 40 mg Lamivudin und 40 bis 60 mg Nevirapin, bevorzugt 6 mg Stavudin, 30 mg Lamivudin und 50 mg Nevirapin vorliegt, und worin das Stavudin vom Nevirapin und Lamivudin im Arzneimittel getrennt ist.

16. Verwendung von Stavudin, Lamivudin und Nevirapin in der Herstellung eines Arzneimittels zur Behandlung eines an einer Virusinfektion leidenden Kindes und mit einem Gewicht im Bereich von 8 bis unter 10 kg, worin das Arzneimittel in der Form einer einzelnen Dosiereinheit mit 8 bis 10 mg Stavudin, 35 bis 55 mg Lamivudin und 65 bis 85 mg Nevirapin, bevorzugt 9 mg Stavudin, 45 mg Lamivudin und 75 mg Nevirapin vorliegt, und worin das Stavudin vom Nevirapin und Lamivudin im Arzneimittel getrennt ist.

17. Verwendung von Stavudin, Lamivudin und Nevirapin in der Herstellung eines Arzneimittels zur Behandlung eines an einer Virusinfektion leidenden Kindes und mit einem Gewicht im Bereich von 10 bis unter 15 kg, worin das Arzneimittel in der Form einer einzelnen Dosiereinheit mit 11 bis 13 mg Stavudin, 50 bis 70 mg Lamivudin und 90 bis 110 mg Nevirapin, bevorzugt 12 mg Stavudin, 60 mg Lamivudin und 100 mg Nevirapin vorliegt, und worin das Stavudin vom Nevirapin und Lamivudin im Arzneimittel getrennt ist.

18. Verwendung von Stavudin, Lamivudin und Nevirapin in der Herstellung eines Arzneimittels zur Behandlung eines an einer Virusinfektion leidenden Kindes und mit einem Gewicht im Bereich von 15 bis unter 20 kg, worin das Arzneimittel in der Form einer einzelnen Dosiereinheit mit 14 bis 16 mg Stavudin, 65 bis 85 mg Lamivudin und 115 bis 135 mg Nevirapin, bevorzugt 15 mg Stavudin, 75 mg Lamivudin und 125 mg Nevirapin vorliegt, und worin das Stavudin vom Nevirapin und Lamivudin im Arzneimittel getrennt ist.

19. Verwendung von Stavudin, Lamivudin und Nevirapin in der Herstellung eines Arzneimittels zur Behandlung eines an einer Virusinfektion leidenden Kindes und mit einem Gewicht im Bereich von 20 bis 29 kg, worin das Arzneimittel in der Form einer einzelnen Dosiereinheit mit 17 bis 19 mg Stavudin, 80 bis 100 mg Lamivudin und 140 bis 160 mg Nevirapin, bevorzugt 18 mg Stavudin, 90 mg Lamivudin und 150 mg Nevirapin vorliegt, und worin das Stavudin vom Nevirapin und Lamivudin im Arzneimittel getrennt ist.

20. Verwendung von Stavudin, Lamivudin und Nevirapin in der Herstellung eines Arzneimittels zur Behandlung eines an einer Virusinfektion leidenden Kindes und mit einem Gewicht im Bereich von 20 bis 24 kg, worin das Arzneimittel in der Form einer einzelnen Dosiereinheit mit 18 mg Stavudin, 90 mg Lamivudin und 150 mg Nevirapin vorliegt, und worin das Stavudin vom Nevirapin und Lamivudin im Arzneimittel getrennt ist.

21. Verwendung von Stavudin, Lamivudin und Nevirapin in der Herstellung eines Arzneimittels zur Behandlung eines an einer Virusinfektion leidenden Kindes und mit einem Gewicht im Bereich von 25 bis 29 kg, worin das Arzneimittel in der Form einer einzelnen Dosiereinheit mit 23 bis 25 mg Stavudin, 110 bis 130 mg Lamivudin und 190 bis 210 mg Nevirapin, bevorzugt 24 mg Stavudin, 120 mg Lamivudin und 200 mg Nevirapin vorliegt, und worin das Stavudin vom Nevirapin und Lamivudin im Arzneimittel getrennt ist.

22. Verwendung nach einem der Ansprüche 16 bis 21, worin die Virusinfektion eine Retrovirusinfektion, wie zum Beispiel eine HIV-Infektion ist.

23. Pharmazeutische Zusammensetzung in der Form einer oralen Einheitsdosierungsform, umfassend Stavudin, Lamivudin und Nevirapin, worin das Lamivudin und Nevirapin in einer Schicht der oralen Dosierungsform bereitgestellt sind und das Stavudin in einer getrennten Schicht bereitgestellt ist.

24. Pharmazeutische Zusammensetzung in der Form einer oralen Einheitsdosierungsform, umfassend Stavudin, Lamivudin und Nevirapin, worin das Stavudin in der Form von Partikeln bereitstellt ist, die mit einem Material zur Verhinderung des Kontakts zwischen dem Stavudin und dem Nevirapin und Lamivudin beschichtet ist.

## Revendications

1. Composition pharmaceutique comprenant une unité de dose unique qui contient de la lamivudine, de la stavudine et de la névirapine, ou des sels ou esters pharmaceutiquement acceptables de ces substances, comprenant de 10 à 120 mg de lamivudine, de 1 à 30 mg de stavudine et de 25 à 170 mg de névirapine, ladite stavudine étant séparée desdites névirapine et lamivudine dans la composition.

2. Composition pharmaceutique selon la revendication 1, comprenant de 10 à 100 mg de lamivudine, de 1 à 25 mg de stavudine et de 25 à 170 mg de névirapine, de préférence 60 mg de lamivudine, 12 mg de stavudine et 100 mg de névirapine, plus préférentiellement 30 mg de lamivudine, 6 mg de stavudine et 50 mg de névirapine

3. Composition pharmaceutique selon la revendication 1 ou la revendication 2, dans laquelle le pourcentage en poids de la névirapine dans la composition est égal à 0,75 à 2,0 fois le pourcentage en poids de la lamivudine dans la composition, et/ou le pourcentage en poids de la lamivudine dans la composition est égal à 2 à 6 fois le pourcentage en poids de la stavudine dans la composition.

4. Composition pharmaceutique selon la revendication 3, dans laquelle ladite forme de dose est une forme de dose à usage oral, de préférence une forme de dose solide.

5. Composition pharmaceutique selon la revendication 4, dans laquelle il est prévu une barrière entre ladite stavudine et lesdites névirapine et lamivudine.

6. Composition pharmaceutique selon la revendication 4 ou la revendication 5, dans laquelle ladite stavudine est présente sous la forme de particules revêtues d'un matériau qui empêche le contact entre ladite stavudine et lesdites névirapine et lamivudine.

7. Composition pharmaceutique selon la revendication 4 ou la revendication 5, comprenant deux couches, dans laquelle la première couche contient lesdites névirapine et lamivudine en combinaison avec un véhicule pharmaceutiquement acceptable, et la deuxième couche contient ladite stavudine en combinaison avec un véhicule pharmaceutiquement acceptable.

8. Composition pharmaceutique selon la revendication 4 ou la revendication 5, comprenant trois couches, dans laquelle la première couche contient lesdites névirapine et lamivudine en combinaison avec un véhicule pharmaceutiquement acceptable, la deuxième couche contient ladite stavudine en combinaison avec un véhicule pharmaceutiquement acceptable, et la troisième couche est une couche inerte contenant au moins un excipient pharmaceutiquement acceptable, la troisième couche étant disposée entre la première et la deuxième couche.

9. Composition pharmaceutique selon la revendication 4 ou la revendication 5, comprenant un noyau et une couche extérieure entourant le noyau, dans laquelle le noyau contient lesdites névirapine et lamivudine en combinaison avec un véhicule pharmaceutiquement acceptable, et la couche extérieure contient ladite stavudine en combinaison avec un véhicule pharmaceutiquement acceptable.

10. Composition pharmaceutique selon la revendication 4 ou la revendication 5, comprenant un noyau et une couche extérieure entourant le noyau, dans laquelle le noyau contient ladite stavudine en combinaison avec un véhicule pharmaceutiquement acceptable, et la couche extérieure contient lesdites névirapine et lamivudine en combinaison avec un véhicule pharmaceutiquement acceptable.

11. Composition pharmaceutique selon l'une quelconque des revendications 4 à 10, dans laquelle ladite forme de dose est une forme de dose dispersible.

12. Utilisation de lamivudine, de névirapine et de stavudine pour fabriquer un médicament destiné au traitement d'une infection virale chez un sujet humain, dans laquelle ledit médicament se présente sous la forme d'une unité de dose unique contenant de 10 à 120 mg de lamivudine, de 1 à 30 mg de stavudine et de 25 à 170 mg de névirapine, et dans laquelle ladite stavudine est séparée desdites névirapine et lamivudine dans le médicament.

13. Utilisation selon la revendication 12 pour traiter un sujet humain âgé de moins de 16 ans.

14. Utilisation de stavudine, de lamivudine et de névirapine pour fabriquer un médicament destiné à traiter un enfant atteint d'une infection virale et ayant une masse corporelle dans la plage de 3 à moins de 6 kg, dans laquelle le médicament se présente sous la forme d'une unité de dose unique contenant de 2 à 4 mg de stavudine, de 10 à 20 mg de lamivudine et de 20 à 30 mg de névirapine, de préférence 3 mg de stavudine, 15 mg de lamivudine et 25 mg de névirapine, et dans laquelle ladite stavudine est séparée desdites névirapine et lamivudine dans le médicament.

15. Utilisation de stavudine, de lamivudine et de névirapine pour fabriquer un médicament destiné à traiter un enfant atteint d'une infection virale et ayant une masse corporelle dans la plage de 6 à moins de 8 kg, dans laquelle le médicament se présente sous la forme d'une unité de dose unique contenant de 5 à 7 mg de stavudine, de 20 à 40 mg de lamivudine et de 40 à 60 mg de névirapine, de préférence 6 mg de stavudine, 30 mg de lamivudine et 50 mg de névirapine, et dans laquelle ladite stavudine est séparée desdites névirapine et lamivudine dans le médicament.

16. Utilisation de stavudine, de lamivudine et de névirapine pour fabriquer un médicament destiné à traiter un enfant atteint d'une infection virale et ayant une masse corporelle dans la plage de 8 à moins de 10 kg, dans laquelle le médicament se présente sous la forme d'une unité de dose unique contenant de 8 à 10 mg de stavudine, de 35 à 55 mg de lamivudine et de 65 à 85 mg de névirapine, de préférence 9 mg de stavudine, 45 mg de lamivudine et 75 mg de névirapine, et dans laquelle ladite stavudine est séparée desdites névirapine et lamivudine dans le médicament.

17. Utilisation de stavudine, de lamivudine et de névirapine pour fabriquer un médicament destiné à traiter un enfant atteint d'une infection virale et ayant une masse corporelle dans la plage de 10 à moins de 15 kg, dans laquelle le médicament se présente sous la forme d'une unité de dose unique contenant de 11 à 13 mg de stavudine, de 50 à 70 mg de lamivudine et de 90 à 110 mg de névirapine, de préférence 12 mg de stavudine, 60 mg de lamivudine et 100 mg de névirapine, et dans laquelle ladite stavudine est séparée desdites névirapine et lamivudine dans le médicament

18. Utilisation de stavudine, de lamivudine et de névirapine pour fabriquer un médicament destiné à traiter un enfant atteint d'une infection virale et ayant une masse corporelle dans la plage de 15 à moins de 20 kg, dans laquelle le médicament se présente sous la forme d'une unité de dose unique contenant de 14 à 16 mg de stavudine, de 65 à 85 mg de lamivudine et de 115 à 135 mg de névirapine, de préférence 15 mg de stavudine, 75 mg de lamivudine et 125 mg de névirapine, et dans laquelle ladite stavudine est séparée desdites névirapine et lamivudine dans le médicament

19. Utilisation de stavudine, de lamivudine et de névirapine pour fabriquer un médicament destiné à traiter un enfant atteint d'une infection virale et ayant une masse corporelle dans la plage de 20 à 29 kg, dans laquelle le médicament se présente sous la forme d'une unité de dose unique contenant de 17 à 19 mg de stavudine, de 80 à 100 mg de lamivudine et de 140 à 160 mg de névirapine, de préférence 18 mg de stavudine, 90 mg de lamivudine et 150 mg de névirapine, et dans laquelle ladite stavudine est séparée desdites névirapine et lamivudine dans le médicament.

20. Utilisation de stavudine, de lamivudine et de névirapine pour fabriquer un médicament destiné à traiter un enfant atteint d'une infection virale et ayant une masse corporelle dans la plage de 20 à 24 kg, dans laquelle le médicament se présente sous la forme d'une unité de dose unique contenant 18 mg de stavudine, 90 mg de lamivudine et 150 mg de névirapine, et dans laquelle ladite stavudine est séparée desdites névirapine et lamivudine dans le médicament.

21. Utilisation de stavudine, de lamivudine et de névirapine pour fabriquer un médicament destiné à traiter un enfant atteint d'une infection virale et ayant une masse corporelle dans la plage de 25 à 29 kg, dans laquelle le médicament se présente sous la forme d'une unité de dose unique contenant de 23 à 25 mg de stavudine, de 110 à 130 mg de lamivudine et de 190 à 210 mg de névirapine, de préférence 24 mg de stavudine, 120 mg de lamivudine et 200 mg de névirapine, et dans laquelle ladite stavudine est séparée desdites névirapine et lamivudine dans le médicament.

22. Utilisation selon l'une quelconque des revendications 16 à 21, dans laquelle l'infection virale est une infection rétrovirale telle qu'une infection par le VIH.

23. Composition pharmaceutique sous la forme d'une forme de dose unitaire à usage oral comprenant de la stavudine, de la lamivudine et de la névirapine, dans laquelle la lamivudine et la névirapine sont présentes dans une couche de la forme de dose à usage oral et la stavudine est présente dans une couche séparée.

24. Composition pharmaceutique sous la forme d'une forme de dose unitaire à usage oral comprenant de la stavudine, de la lamivudine et de la névirapine, dans laquelle ladite stavudine est présente sous la forme de particules revêtues d'un matériau qui empêche le contact entre ladite stavudine et lesdites névirapine et lamivudine.
